# EUROPEAN PATENT SPECIFICATION

(11) **EP 2 773 773 B1**
(45) Date of publication and mention of the grant of the patent: **11.01.2017**
(21) Application number: 12844825.5
(22) Date of filing: 25.10.2012
(51) Int. Cl.: C12Q 1/68, C12N 15/12, C07K 16/18, A61K 39/395

(54) **T-CELL RECEPTOR CLONOTYPES SHARED AMONG ANKYLOSING SPONDYLITIS PATIENTS**
VON MEHREREN SPONDYLITIS-ANKYLOSANS-PATIENTEN GETEILTE T-ZELL-REZEPTOR-KLONOTYPEN
CLONOTYPES DE RÉCEPTEURS DE L'ANTIGÈNE DES LYMPHOCYTES T PARTAGÉS ENTRE DES PATIENTS ATTEINTS DE SPONDYLARTHRITE ANKYLOSANTE

(30) Priority: 04.11.2011 US 201161556125 P; 17.11.2011 US 201161561234 P
(43) Date of publication of application: 10.09.2014
(73) Proprietor: Adaptive Biotechnologies Corporation, Seattle, Washington 98102 (US)
(72) Inventor: FAHAM, Malek, South San Francisco, CA 94080 (US); CARLTON, Victoria, South San Francisco, CA 94080 (US); MOORHEAD, Martin, South San Francisco, CA 94080 (US); ZHENG, Jianbiao, South San Francisco, CA 94080 (US); ASBURY, Thomas, South San Francisco, CA 94080 (US)
(74) Representative: Boult Wade Tennant
(86) International application number: PCT/US2012/061977
(87) International publication number: WO 2013/066726

(56) References cited:
- WO-A1-97/46706
- WO-A2-2011/083996
- US-A- 5 667 967
- US-A1- 2011 207 134
- US-A1- 2011 251 099
- BAUM P D ET AL: "Direct measurement of T-cell receptor repertoire diversity with AmpliCot", NATURE METHODS, NATURE PUBLISHING GROUP, GB, vol. 3, no. 11, 1 November 2006 (2006-11-01), pages 895-901, XP002581658, ISSN: 1548-7091, DOI: 10.1038/NMETH949 [retrieved on 2006-10-23]
- DOUGLAS FREEMAN J ET AL: "Profiling the T-cell receptor beta-chain repertoire by massively parallel sequencing", GENOME RESEARCH, COLD SPRING HARBOR LABORATORY PRESS, vol. 19, no. 10, 1 October 2009 (2009-10-01), pages 1817-1824, XP002636496, ISSN: 1088-9051, DOI: 10.1101/GR.092924.109 [retrieved on 2009-06-18]

### SEQUENCE LISTING

<110> Sequenta, Inc.
   Faham, Malek
   Carlton, Victoria
   Moorhead, Martin
   Zheng, Jianbiao
   Asbury, Thomas
<120> T-CELL RECEPTOR CLONOTYPES SHARED AMONG **ANKYLOSING** SPONDYLITIS PATIENTS
<130> 820WO00
<150> 61/556125
   <151> 2011-11-04
<150> 61/561234
   <151> 2011-11-17
<160> 2
<170> PatentIn version 3.5
<210> 1
   <211> 26
   <212> PRT
   <213> Homo sapiens
<400> 1
   Leu Cys Ala Ser Ser Leu Glu Ala Ser Gly Ser Ser Tyr Asn Glu Gln
   1               5                   10                  15
   Phe Phe Gly Pro Gly Thr Arg Leu Thr Val
               20                  25
<210> 2
   <211> 26
   <212> PRT
   <213> Homo sapiens
<400> 2
   Val Tyr Phe Cys Ala Ser Ser Asp Ser Ser Gly Ser Thr Asp Thr Gln
   1               5                   10                  15
   Tyr Phe Gly Pro Gly Thr Arg Leu Thr Val
               20                  25

## Description

### BACKGROUND OF THE INVENTION

Ankylosing spondylitis (AS, from Greek ankylos, bent; spondylos, vertebrae), previously known as Bechterew's disease, Bechterew syndrome, and Marie Strümpell disease, a form of Spondyloarthritis, is a chronic, inflammatory arthritis and autoimmune disease. It mainly affects joints in the spine and the sacroilium in the pelvis, causing eventual fusion of the spine. It is a member of the group of the spondyloarthropathies with a strong genetic predisposition. Complete fusion results in a complete rigidity of the spine, a condition known as bamboo spine.

The typical patient is a young male, aged 18-30, when symptoms of the disease first appear, with chronic pain and stiffness in the lower part of the spine or sometimes the entire spine, often with pain referred to one or other buttock or the back of thigh from the sacroiliac joint. Men are affected more than women by a ratio about of 3:1, with the disease usually taking a more painful course in men than women. In 40% of cases, ankylosing spondylitis is associated with an inflammation of the eye (iridocyclitis and uveitis), causing redness, eye pain, vision loss, floaters and photophobia. Another common symptom is generalized fatigue and sometimes nausea. Less commonly aortitis, apical lung fibrosis and ectasia of the sacral nerve root sheaths may occur. As with all the seronegative spondyloarthropathies, lifting of the nails (onycholysis) may occur.

There is no direct test to diagnose AS. A clinical examination and X-ray studies of the spine, which show characteristic spinal changes and sacroiliitis, are the major diagnostic tools. A drawback of X-ray diagnosis is that signs and symptoms of AS have usually been established as long as 8-10 years prior to X-ray-evident changes occurring on a plain film X-ray, which means a delay of as long as 10 years before adequate therapies can be introduced. Options for earlier diagnosis are tomography and magnetic resonance imaging of the sacroiliac joints, but the reliability of these tests is still unclear. The Schober's test is a useful clinical measure of flexion of the lumbar spine performed during examination.

During acute inflammatory periods, AS patients will sometimes show an increase in the blood concentration of C-reactive protein (CRP) and an increase in the erythrocyte sedimentation rate (ESR), but there are many with AS whose CRP and ESR rates do not increase so normal CRP and ESR results do not always correspond with the amount of inflammation a person actually has. Sometimes people with AS have normal level results, yet are experiencing a significant amount of inflammation in their bodies.

There are three major types of medications used to treat ankylosing spondylitis:1) Anti-inflammatory drugs, which include NSAIDs such as ibuprofen, phenylbutazone, indomethacin, naproxen and COX-2 inhibitors, which reduce inflammation and pain Opioid analgesics have also been proven by clinical evidence to be very effective in alleviating the type of chronic pain commonly experienced by those suffering from AS, especially in time-release formulations; 2) DMARDs such as ciclosporin, methotrexate, sulfasalazine, and corticosteroids, used to reduce the immune system response through immunosuppression; 3) TNFα blockers (antagonists) such as etanercept, infliximab and adalimumab (also known as biologics), are indicated for the treatment of and are effective immunosuppressants in as in other autoimmune diseases.

TNFα blockers have been shown to be the most promising treatment, slowing the progress of AS in the majority of clinical cases, helping many patients receive a significant reduction, though not elimination, of their inflammation and pain. They have also been shown to be highly effective in treating not only the arthritis of the joints but also the spinal arthritis associated with AS. A drawback, besides the often high cost, is the fact that these drugs increase the risk of infections. For this reason, the protocol for any of the TNF-α blockers include a test for tuberculosis (like Mantoux or Heaf) before starting treatment. In case of recurrent infections, even recurrent sore throats, the therapy may be suspended because of the involved immunosuppression. Patients taking the TNF medications are advised to limit their exposure to others who are or may be carrying a virus (such as a cold or influenza) or who may have a bacterial or fungal infection.

AS affects produces symptoms that are very common in the healthy populations. For example, a patient presenting complaining of severe back pain need not be experiencing an AS flare but rather might just have routine back pain. The physician is forced to make a decision about whether to treat these symptoms with expensive drugs with potentially severe side effects without a very precise view into the state of the disease. CRP and ESR do not provide a very precise view of the disease status. At the same time the course of the untreated disease can result in debilitating long term spinal damage. This state of affairs leads to a difficult clinical challenge and significant overtreatment is used. The availability of an objective measure that reflects disease activity can be of great help in the management of AS patients.

Profiles of nucleic acids encoding immune molecules, such as T cell or B cell receptors, or their components, contain a wealth of information on the state of health or disease of an organism, so that the use of such profiles as diagnostic or prognostic indicators has been proposed for a wide variety of conditions, including autoimmune conditions e.g. Faham and Willis, U.S. patent publication 2010/0151471 and 2011/0207134; Freeman et al, Genome Research, 19: 1817-1824 (2009); Boyd et al, Sci. Transl. Med., 1(12): 12ra23 (2009); He et al, Oncotarget (March 8, 2011). Such sequence-based profiles are capable of much greater sensitivity than approaches based on size distributions of amplified CDR-encoding regions, sequence sampling by microarrays, hybridization kinetics curves from PCR amplicons, or other approaches, e.g., Morley et al, U.S. patent 5,418,134; van Dongen et al, Leukemia, 17: 2257-2317 (2003); Ogle et al, Nucleic Acids Research, 31: e139 (2003); Wang et al, BMC Genomics, 8: 329 (2007); Baum et al, Nature Methods, 3(11): 895-901 (2006).

In view of the personal and social impact of AS, it would be highly desirable if measures of disease activity were available based on immune sequence profiles that could readily be correlated to states of health or disease and/or likelihood of treatment success.

### SUMMARY OF THE INVENTION

The present invention is drawn to methods for determining the disease status of ankylosing spondylitis patients by analysis of sequence-based clonotype profiles of patient T-cell receptor β chains. The invention is exemplified in a number of implementations and applications, some of which are summarized below and throughout the specification.

In one aspect the invention includes a method for determining a disease status of a patient suffering from, or suspected of suffering from, ankylosing spondylitis comprising the steps of (i) determining in a clonotype profile of a tissue sample of the patient the presence, absence and/or quantity of clonotypes encoding segments of a T-cell receptor at least seventy percent homologous to a segment in the group consisting of LCASSLEASGSSYNEQFFGPGTRLTV (SEQ ID NO: 1) (peptide 1) and VYFCASSDSSGSTDTQYFGPGTRLTV (SEQ ID NO: 2) (peptide 2); and (ii) correlating the presence, absence and/or quantity of such clonotypes to a status of ankylosing spondylitis in the patient. In some embodiments, such methods comprise the steps of of (i) determining in a clonotype profile of a tissue sample of the patient the presence and/or quantity of clonotypes encoding segments of a T-cell receptor at least ninety percent homologous to a segment in the group consisting of LCASSLEASGSSYNEQFFGPGTRLTV (SEQ ID NO: 1) (peptide 1) and VYFCASSDSSGSTDTQYFGPGTRLTV (SEQ ID NO: 2) (peptide 2); (ii) correlating the presence and/or quantity of such clonotypes to a status of ankylosing spondylitis in the patient; and (iii) treating the patient with a medication for ameliorating effects of ankylosing spondylitis.

In another aspect the invention includes a method of treating a patient with ankylosing spondylitis by delivering an effective amount of an antibody specific for an amino acid segment of a T cell receptor, the amino acid segment being selected from the group consisting of LCASSLEASGSSYNEQFFGPGTRLTV (SEQ ID NO: 1) and any 6 to 20 amino acid segment thereof and VYFCASSDSSGSTDTQYFGPGTRLTV (SEQ ID NO: 2) and any 6 to 20 amino acid segment thereof.

These above-characterized aspects, as well as other aspects, of the present invention are exemplified in a number of illustrated implementations and applications, some of which are shown in the figures and characterized in the claims section that follows. However, the above summary is not intended to describe each illustrated embodiment or every implementation of the invention.

### BRIEF DESCRIPTION OF THE DRAWINGS

The novel features of the invention are set forth with particularity in the appended claims. A better understanding of the features and advantages of the present invention is obtained by reference to the following detailed description that sets forth illustrative embodiments, in which the principles of the invention are utilized, and the accompanying drawings of which:

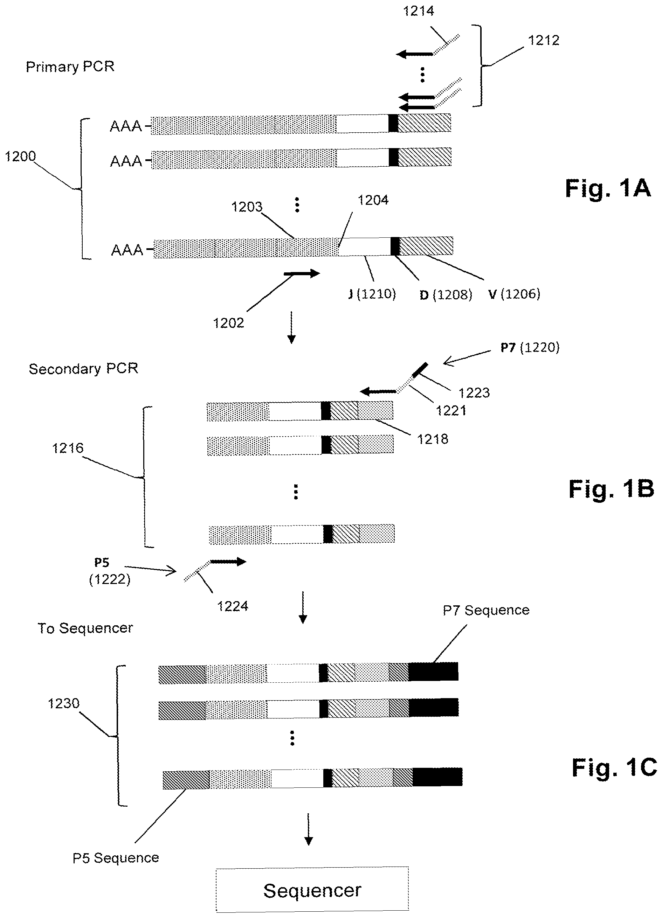

FIGS. 1A-1C show a two-staged PCR scheme for amplifying TCRβ genes.

### DETAILED DESCRIPTION OF THE INVENTION

The practice of the present invention may employ, unless otherwise indicated, conventional techniques and descriptions of molecular biology (including recombinant techniques), bioinformatics, cell biology, and biochemistry, which are within the skill of the art. Such conventional techniques include, but are not limited to, sampling and analysis of blood cells, nucleic acid sequencing and analysis, constructing and applying immunoassays, and the like. Specific illustrations of suitable techniques can be had by reference to the example herein below. However, other equivalent conventional procedures can, of course, also be used.

The invention is directed to methods for determining the disease status of patients who are or may be suffering from ankylosing spondylitis. In one aspect, such determination is made by detecting the presence or absence or quantity of T-cell receptor beta (TCRβ) clonotypes that encode TCRβ segments at least seventy percent homologous to either of the segments of the group consisting of LCASSLEASGSSYNEQFFGPGTRLTV (SEQ ID NO: 1) and
VYFCASSDSSGSTDTQYFGPGTRLTV (SEQ ID NO: 2). In another embodiment, such detection is for clonotypes encoding TCRβ segments at least eighty percent homologous to either of the segments in the above group. In another embodiment, such detection is for clonotypes encoding TCRβ segments at least ninety percent homologous to either of the segments in the above group. In another embodiment, such detection is for clonotypes encoding TCRβ segments identical to either of the segments in the above group. As used herein, the term "AS-related peptides" means the peptides
LCASSLEASGSSYNEQFFGPGTRLTV (SEQ ID NO: 1) and VYFCASSDSSGSTDTQYFGPGTRLTV (SEQ ID NO: 2). In one embodiment of the invention such clonotypes are assayed by generating a sequence-based clonotype profile from a tissue sample from a patient, for example, using the process disclosed by Faham and Willis, U.S. patent publication 2011/020713 4. Briefly, in one aspect, a sequence-based clonotype profile of an individual is obtained and the method of the invention implemented using the following steps: (a) obtaining a nucleic acid sample from T-cells of the individual; (b) spatially isolating individual molecules derived from such nucleic acid sample; (c) sequencing said spatially isolated individual molecules; (d) determining abundances of different sequences of the nucleic acid molecules from the nucleic acid sample to generate the clonotype profile; and (e) determining the presence, absence and/or quantity of clonotypes encoding segments of a T-cell receptor at least seventy percent homologous to a segment in the group consisting of LCASSLEASGSSYNEQFFGPGTRLTV (SEQ ID NO: 1) and
VYFCASSDSSGSTDTQYFGPGTRLTV (SEQ ID NO: 2). In some embodiments, the step of determining includes determining the presence, absence and/or quantity of clonotypes encoding segments of a T-cell receptor at least eighty percent or ninety percent homologous to the above segments, or identical to the above segments. In still other embodiments, the method may be implemented by the following steps: (a) obtaining a sample from a patient comprising T-cells; (b) amplifying molecules of nucleic acid from the T-cells of the sample, the molecules of nucleic acid comprising recombined DNA sequences from T-cell receptor genes; (c) sequencing the amplified molecules of nucleic acid to form a clonotype profile; and (d) determining the presence, absence and/or quantity of clonotypes encoding segments of a T-cell receptor at least seventy percent homologous to a segment in the group consisting of LCASSLEASGSSYNEQFFGPGTRLTV (SEQ ID NO: 1) and
VYFCASSDSSGSTDTQYFGPGTRLTV (SEQ ID NO: 2). As above, other embodiments may call for determining segments with differing homologies to the above sequences. In some embodiments, clonotype profiles include every clonotype present at a frequency of .01 percent or greater with a probability of ninety-nine percent. In other embodiments, clonotype profiles include at least 10⁴ clonotypes, or at least 10⁵ clonotypes.

In another embodiment, the step of sequencing comprises bidirectionally sequencing each of the spatially isolated individual molecules to produce at least one forward sequence read and at least one reverse sequence read. Further to the latter embodiment, at least one of the forward sequence reads and at least one of the reverse sequence reads have an overlap region such that bases of such overlap region are determined by a reverse complementary relationship between such sequence reads. In still another embodiment, each of the somatically rearranged regions comprise a V region and a J region and the step of sequencing further includes determining a sequence of each of the individual nucleic acid molecules from one or more of its forward sequence reads and at least one reverse sequence read starting from a position in a J region and extending in the direction of its associated V region.

A sample from a patient may be from a variety of tissues, but usually a sample is a blood sample. From the sample RNA is extracted using conventional techniques as the source of nucleic acids amplified and processed in accordance with Faham and Willis (cited above).

In another aspect of the invention, the presence, absence and/or quantity of the TCRβ segments may be detected or measured by an immunoassay using one or more antibodies specific for peptides 6 to 25 amino acids in length derived from contiguous segment of LCASSLEASGSSYNEQFFGPGTRLTV (SEQ ID NO: 1) or VYFCASSDSSGSTDTQYFGPGTRLTV (SEQ ID NO: 2). Guidance for constructing immunoassays is found in many treatises, including Wild, Editor, The Immunoassay Handbook, Third Edition (Elsevier Science, 2005). Guidance for making peptide-specific antibodies is found in U.S. patent 5,231,01 2. Antibodies specific for the
above segments may also be used to detect and quantify by flow cytometry T cells having TCRs with the segments, e.g., Thiel et al, Clinical Immunology, 111(2): 155-161 (2004); Gratama et al, Cytometry part A, 58A: 79-86 (2004); Sims et al, Expert Reviews of Vaccines, 9(7): 765-774 (2010); and the like.

In another aspect of the invention, antibodies specific for TCRβs with the above segments may be used to inhibit the function of T cells carrying such receptors, including but not limited to autoimmune-related effects of such T cells, such as AS-related effects. In this aspect of the invention, an effective amount of a therapeutic antibody specific for peptide LCASSLEASGSSYNEQFFGPGTRLTV (SEQ ID NO: 1) or a 6-20 amino acid segment thereof or VYFCASSDSSGSTDTQYFGPGTRLTV (SEQ ID NO: 2) or a 6-20 amino acid segment therof is administered to a patient suffering from AS.

### Samples

Clonotype profiles for use with methods of the invention are obtained from samples of T cells, which are present in a wide variety of tissues. T-cells include helper T cells (effector T cells or Th cells), cytotoxic T cells (CTLs), memory T cells, and regulatory T cells, which may be distinguished by cell surface markers. In one aspect a sample of T cells includes at least 1,000 T cells; but more typically, a sample includes at least 10,000 T cells, and more typically, at least 100,000 T cells. In another aspect, a sample includes a number of T cells in the range of from 1000 to 1,000,000 cells.

Samples (sometimes referred to as "tissue samples") used in the methods of the invention can come from a variety of tissues, including, for example, blood and blood plasma, lymph fluid, cerebrospinal fluid surrounding the brain and the spinal cord, synovial fluid surrounding bone joints, and the like. In one embodiment, the sample is a blood sample. The blood sample can be about 0.1, 0.2, 0.3, 0.4, 0.5, 0.6, 0.7, 0.8, 0.9, 1.0, 1.5, 2.0, 2.5, 3.0, 3.5, 4.0, 4.5, or 5.0 mL.

A sample or tissue sample includes nucleic acid, for example, DNA (e.g., genomic DNA) or RNA (e.g., messenger RNA). The nucleic acid can be cell-free DNA or RNA, e.g. extracted from the circulatory system, Vlassov et al, Curr. Mol. Med., 10: 142-165 (2010); Swarup et al, FEBS Lett., 581: 795-799 (2007). In the methods of the invention, the amount of RNA or DNA from a subject that can be analyzed varies widely. For generating a clonotype profile, sufficient nucleic acid must be in a sample to obtain a useful representation of an individual's TCR repertoire. More particularly, for generating a clonotype profile from genomic DNA at least 1 ng of total DNA from T cells (i.e. about 300 diploid genome equivalents) is extracted from a sample; in another embodiment, at least 2 ng of total DNA (i.e. about 600 diploid genome equivalents) is extracted from a sample; and in another embodiment, at least 3 ng of total DNA (i.e. about 900 diploid genome equivalents) is extracted from a sample. One of ordinary skill would recognize that as the fraction of lymphocytes in a sample decreases, the foregoing minimal amounts of DNA must increase in order to generate a clonotype profile containing more than about 1000 independent clonotypes. For generating a clonotype profile from RNA, in one embodiment, a sufficient amount of RNA is extracted so that at least 1000 transcripts are obtained which encode distinct TCRs, or fragments thereof. The amount of RNA that corresponds to this limit varies widely from sample to sample depending on the fraction of lymphocytes in a sample, developmental stage of the lymphocytes, and the like. In one embodiment, at least 100 ng of RNA is extracted from a tissue sample containing T cells for the generating of a clonotype profile; in another embodiment, at least 500 ng of RNA is extracted from a tissue sample containing T cells for the generating of a clonotype profile. RNA used in methods of the invention may be either total RNA extracted from a tissue sample or polyA RNA extracted directly from a tissue sample or from total RNA extracted from a tissue sample. The above nucleic acid extractions may be carried out using commercially available kits, e.g. from Invitrogen (Carlsbad, CA), Qiagen (San Diego, CA), or like vendors. Guidance for extracting RNA is found in Liedtke et al., PCR Methods and Applications, 4: 185-187 (1994); and like references.

In some embodiments, a sample containing lymphocytes is sufficiently large so that substantially every T cell with a distinct clonotype is represented therein, thereby forming a repertoire (as the term is used herein). In one embodiment, a sample is taken that contains with a probability of ninety-nine percent every clonotype of a population present at a frequency of .001 percent or greater. In another embodiment, a sample is taken that contains with a probability of ninety-nine percent every clonotype of a population present at a frequency of .0001 percent or greater. In one embodiment, a sample of T cells includes at least a half million cells, and in another embodiment such sample includes at least one million cells.

Whenever a source of material from which a sample is taken is scarce, such as, clinical study samples, or the like, DNA from the material may be amplified by a non-biasing technique, such as whole genome amplification (WGA), multiple displacement amplification (MDA); or like technique, e.g. Hawkins et al, Curr. Opin. Biotech., 13: 65-67 (2002); Dean et al, Genome Research, 11: 1095-1099 (2001); Wang et al, Nucleic Acids Research, 32: e76 (2004); Hosono et al, Genome Research, 13: 954-964 (2003); and the like.

Blood samples are of particular interest and may be obtained using conventional techniques, e.g. Innis et al, editors, PCR Protocols (Academic Press, 1990); or the like. For example, white blood cells may be separated from blood samples using convention techniques, e.g. RosetteSep kit (Stem Cell Technologies, Vancouver, Canada). Likewise, other fractions of whole blood, such as peripheral blood mononuclear cells (PBMCs) may be isolated for use with methods of the invention using commercially available kits, (e.g. Miltenyi Biotec, Auburn, CA), or the like. Blood samples may range in volume from 100 µL to 10 mL; in one aspect, blood sample volumes are in the range of from 200 µL to 2 mL. DNA and/or RNA may then be extracted from such blood sample using conventional techniques for use in methods of the invention, e.g. DNeasy Blood & Tissue Kit (Qiagen, Valencia, CA). Optionally, subsets of white blood cells, e.g. lymphocytes, may be further isolated using conventional techniques, e.g. fluorescently activated cell sorting (FACS)(Becton Dickinson, San Jose, CA), magnetically activated cell sorting (MACS)(Miltenyi Biotec, Auburn, CA), or the like.

### Antibodies For Treatment and Detection

AS-related peptides or segments thereof may be used to make antibodies for therapeutic or immunoassay applications using conventional peptide antibody techniques, e.g. U.S. patent 5,231,012; U.S. patent 4,474,754; Walter et al, Genetic Engineering, 5: 61-91 (1983), or the like. Briefly, an AS-related peptide or a segment thereof is conjugated to a carrier molecule, cell line to form hybridomas, which are screened for peptide-specific antibodies having desired affinity and specificity. Such antibodies may be further processed, e.g. to improve affinity, specificity, reduce immunogenicity, and the like, by use of known antibody engineering techniques, such as those disclosed in references cited below. Such further processing may include humanization, e.g. as disclosed in U.S. patents 7,892,550 and 8,030, 0.23.

Once B cells from an immunized animal, e.g. a rabbit, are available, hybridomas are produced by well known techniques. Usually, the process involves the fusion of an immortalizing cell line with a B-lymphocyte which produces the desired antibody. Alternatively, non-fusion techniques for generating an immortal antibody producing cell lines are possible, and come within the purview of the present invention, e.g. virally induced transformation: Casali et al., "Human Monoclonals from Antigen-Specific Selection of B Lymphocytes and Transformation by EBV," Science, Vol. 234, pgs. 476-479 (1986). Immortalizing cell lines are usually transformed mammalian cells, particularly myeloma cells of rodent, bovine, and human origin. Most frequently, rat or mouse myeloma cell lines are employed as a matter of the convenience and availability. Techniques for obtaining the appropriate lymphocytes from mammals injected with the target antigen are well known. Generally, either peripheral blood lymphocytes (PBLs) are used if cells of human origin are desired, or spleen cells or lymph node cells are used if non-human mammalian sources are desired. A host mammal is injected with repeated dosages of the purified antigen, and the mammal is permitted to generate the desired antibody producing cells before these are harvested for fusion with the immortalizing cell line. Techniques for fusion are also well known in the art, and in general, involve mixing the cells with a fusing agent, such as polyethylene glycol. Hybridomas are selected by standard procedures, such as HAT selection. From among these hybridomas, those secreting the desired antibody, i.e. specific for the desired peptide, are selected by assaying their culture medium by standard immunoassays, such as Western blotting, ELISA, RIA, CSIF neutralizing capability, or the like. Antibodies are recovered from the medium using standard protein purification techniques, e.g. Tijssen, Practice and Theory of Enzyme Immunoassays (Elsevier, Amsterdam, 1985). Many references are available for guidance in applying any of the above techniques, e.g. Kohler et al., Hybridoma Techniques (Cold Spring Harbor Laboratory, New York, 1980); Tijssen, Practice and Theory of Enzyme Immunoassays (Elsevier, Amsterdam, 1985); Campbell, Monoclonal Antibody Technology (Elsevier, Amsterdam, 1984); Hurrell, Monoclonal Hybridoma Antibodies: Techniques and Applications (CRC Press, Boca Raton, Fla. 1982); and the like. Antibodies and antibody fragments characteristic of hybridomas of the invention can also be produced by recombinant means by extracting messenger RNA, constructing a cDNA library, and selecting clones which encode segments of the antibody molecule, e.g. Huse et al, Science, Vol. 246, pgs. 1275-1281 (1989). Once a nucleotide sequence is available that encodes the variable region of a suitable antibody, properties of such antibody may be improved using conventional techniques, for example as disclosed in the following references: Barbas et al, Proc. Natl. Acad. Sci., 88: 7978-7982 (1991), and pHEN1 and its related family members, e.g. disclosed in Hoogenboom et al, Nucleic Acids Research, 19: 4133-4137 (1991); and U.S. patent 5,969,108; 6,806,079; 7,662,557; and related patents; and Sidhu, editor, Phage Display in Biotechnology and Drug Discovery (CRC Press, 2005); Lutz and Bornscheuer, Editors, Protein Engineering Handbook (Wiley-VCH, 2009); and the like.

Once a therapeutic antibody is obtained, it may be re-engineered and/or manufactured and formulated for treating humans using methods known in the art, e.g. as disclosed in U.S. patents 7,892,550 and 8,030, 023. Usually, a therapeutic antibody is an isolated antibody of the invention which is included in a therapeutic formulation. In one aspect, the invention provides a method of treating ankylosing spondylitis in a subject, said method comprising administering to the subject an effective amount of an antibody of the invention, whereby said condition is treated. In one aspect, the invention provides use of an antibody of the invention in the preparation of a medicament for the therapeutic and/or prophylactic treatment of ankylosing spondylitis.

Therapeutic formulations comprising an antibody of the invention are prepared for storage by mixing the antibody having the desired degree of purity with optional physiologically acceptable carriers, excipients or stabilizers (*Remington's Pharmaceutical Sciences* 16th edition, Osol, A. Ed. (1980)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

The formulation herein may also contain more than one active compound as necessary for the particular indication being treated, preferably those with complementary activities that do not adversely affect each other. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for in vivo administration must be sterile. This is readily accomplished by filtration through sterile filtration membranes.

Sustained-release preparations may be prepared. Suitable examples of sustained-release preparations include semipermeable matrices of solid hydrophobic polymers containing the immunoglobulin of the invention, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated immunoglobulins remain in the body for a long time, they may denature or aggregate as a result of exposure to moisture at 37° C., resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, etc. The containers may be formed from a variety of materials such as glass or plastic. The container holds a composition which is by itself or when combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. Alternatively, or additionally, the article of manufacture may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### Amplification of Nucleic Acid Populations for Clonotype Profiles

Amplicons of target populations of nucleic acids may be generated by a variety of amplification techniques. In one aspect of the invention, multiplex PCR is used to amplify members of a mixture of nucleic acids, particularly mixtures comprising recombined immune molecules such as T cell receptors, or portions thereof. Guidance for carrying out multiplex PCRs of such immune molecules is found in the following references: Morley, U.S. patent 5,296,351; Gorski, U.S. patent 5,837,447; Dau, U.S. patent 6,087,096; Von Dongen et al, U.S. patent publication 2006/0234234; European patent publication EP 1544308B1; and the like.

After amplification of DNA from the genome (or amplification of nucleic acid in the form of cDNA by reverse transcribing RNA), the individual nucleic acid molecules can be isolated, optionally re-amplified, and then sequenced individually. Exemplary amplification protocols may be found in van Dongen et al, Leukemia, 17: 2257-2317 (2003) or van Dongen et al, U.S. patent publication 2006/023423 4. Briefly, an exemplary protocol is as follows:
Reaction buffer: ABI Buffer II or ABI Gold Buffer (Life Technologies, San Diego, CA); 50 µL final reaction volume; 100 ng sample DNA; 10 pmol of each primer (subject to adjustments to balance amplification as described below); dNTPs at 200 µM final concentration; MgCl₂ at 1.5 mM final concentration (subject to optimization depending on target sequences and polymerase); Taq polymerase (1-2 U/tube); cycling conditions: preactivation 7 min at 95°C; annealing at 60°C; cycling times: 30s denaturation; 30s annealing; 30s extension. Polymerases that can be used for amplification in the methods of the invention are commercially available and include, for example, Taq polymerase, AccuPrime polymerase, or Pfu. The choice of polymerase to use can be based on whether fidelity or efficiency is preferred.

Real time PCR, picogreen staining, nanofluidic electrophoresis (e.g. LabChip) or UV absorption measurements can be used in an initial step to judge the functional amount of amplifiable material.

In one aspect, multiplex amplifications are carried out so that relative amounts of sequences in a starting population are substantially the same as those in the amplified population, or amplicon. That is, multiplex amplifications are carried out with minimal amplification bias among member sequences of a sample population. In one embodiment, such relative amounts are substantially the same if each relative amount in an amplicon is within five fold of its value in the starting sample. In another embodiment, such relative amounts are substantially the same if each relative amount in an amplicon is within two fold of its value in the starting sample. As discussed more fully below, amplification bias in PCR may be detected and corrected using conventional techniques so that a set of PCR primers may be selected for a predetermined repertoire that provide unbiased amplification of any sample.

In regard to many repertoires based on TCR or BCR sequences, a multiplex amplification optionally uses all the V segments. The reaction is optimized to attempt to get amplification that maintains the relative abundance of the sequences amplified by different V segment primers. Some of the primers are related, and hence many of the primers may "cross talk," amplifying templates that are not perfectly matched with it. The conditions are optimized so that each template can be amplified in a similar fashion irrespective of which primer amplified it. In other words if there are two templates, then after 1,000 fold amplification both templates can be amplified approximately 1,000 fold, and it does not matter that for one of the templates half of the amplified products carried a different primer because of the cross talk. In subsequent analysis of the sequencing data the primer sequence is eliminated from the analysis, and hence it does not matter what primer is used in the amplification as long as the templates are amplified equally.

In one embodiment, amplification bias may be avoided by carrying out a two-stage amplification (as described in Faham and Willis, cited above) wherein a small number of amplification cycles are implemented in a first, or primary, stage using primers having tails non-complementary with the target sequences. The tails include primer binding sites that are added to the ends of the sequences of the primary amplicon so that such sites are used in a second stage amplification using only a single forward primer and a single reverse primer, thereby eliminating a primary cause of amplification bias. Preferably, the primary PCR will have a small enough number of cycles (e.g. 5-10) to minimize the differential amplification by the different primers. The secondary amplification is done with one pair of primers and hence the issue of differential amplification is minimal. One percent of the primary PCR is taken directly to the secondary PCR. Thirty-five cycles (equivalent to ∼28 cycles without the 100 fold dilution step) used between the two amplifications were sufficient to show a robust amplification irrespective of whether the breakdown of cycles were: one cycle primary and 34 secondary or 25 primary and 10 secondary. Even though ideally doing only 1 cycle in the primary PCR may decrease the amplification bias, there are other considerations. One aspect of this is representation. This plays a role when the starting input amount is not in excess to the number of reads ultimately obtained. For example, if 1,000,000 reads are obtained and starting with 1,000,000 input molecules then taking only representation from 100,000 molecules to the secondary amplification would degrade the precision of estimating the relative abundance of the different species in the original sample. The 100 fold dilution between the 2 steps means that the representation is reduced unless the primary PCR amplification generated significantly more than 100 molecules. This indicates that a minimum 8 cycles (256 fold), but more comfortably 10 cycle (∼1,000 fold), may be used. The alternative to that is to take more than 1% of the primary PCR into the secondary but because of the high concentration of primer used in the primary PCR, a big dilution factor can be used to ensure these primers do not interfere in the amplification and worsen the amplification bias between sequences. Another alternative is to add a purification or enzymatic step to eliminate the primers from the primary PCR to allow a smaller dilution of it. In this example, the primary PCR was 10 cycles and the second 25 cycles.

### Generating Sequence Reads for Clonotypes

Any high-throughput technique for sequencing nucleic acids can be used in the method of the invention. Preferably, such technique has a capability of generating in a cost-effective manner a volume of sequence data from which at least 1000 clonotypes can be determined, and preferably, from which at least 10,000 to 1,000,000 clonotypes can be determined. DNA sequencing techniques include classic dideoxy sequencing reactions (Sanger method) using labeled terminators or primers and gel separation in slab or capillary, sequencing by synthesis using reversibly terminated labeled nucleotides, pyrosequencing, 454 sequencing, allele specific hybridization to a library of labeled oligonucleotide probes, sequencing by synthesis using allele specific hybridization to a library of labeled clones that is followed by ligation, real time monitoring of the incorporation of labeled nucleotides during a polymerization step, polony sequencing, and SOLiD sequencing. Sequencing of the separated molecules has more recently been demonstrated by sequential or single extension reactions using polymerases or ligases as well as by single or sequential differential hybridizations with libraries of probes. These reactions have been performed on many clonal sequences in parallel including demonstrations in current commercial applications of over 100 million sequences in parallel. These sequencing approaches can thus be used to study the repertoire of T-cell receptor (TCR) and/or B-cell receptor (BCR). In one aspect of the invention, high-throughput methods of sequencing are employed that comprise a step of spatially isolating individual molecules on a solid surface where they are sequenced in parallel. Such solid surfaces may include nonporous surfaces (such as in Solexa sequencing, e.g. Bentley et al, Nature,456: 53-59 (2008) or Complete Genomics sequencing, e.g. Drmanac et al, Science, 327: 78-81 (2010)), arrays of wells, which may include bead- or particle-bound templates (such as with 454, e.g. Margulies et al, Nature, 437: 376-380 (2005) or Ion Torrent sequencing, U.S. patent publication 2010/0137143 or 2010/0304982), micromachined membranes (such as with SMRT sequencing, e.g. Eid et al, Science, 323: 133-138 (2009)), or bead arrays (as with SOLiD sequencing or polony sequencing, e.g. Kim et al, Science, 316: 1481-1414 (2007)). In another aspect, such methods comprise amplifying the isolated molecules either before or after they are spatially isolated on a solid surface. Prior amplification may comprise emulsion-based amplification, such as emulsion PCR, or rolling circle amplification. Of particular interest is Solexa-based sequencing where individual template molecules are spatially isolated on a solid surface, after which they are amplified in parallel by bridge PCR to form separate clonal populations, or clusters, and then sequenced, as described in Bentley et al (cited above) and in manufacturer's instructions (e.g. TruSeq™ Sample Preparation Kit and Data Sheet, Illumina, Inc., San Diego, CA, 2010); and further in the following references: U.S. patents 6,090,592; 6,300,070; 7,115,400; and EP0972081B1. In one embodiment, individual molecules disposed and amplified on a solid surface form clusters in a density of at least 10⁵ clusters per cm²; or in a density of at least 5x10⁵ per cm²; or in a density of at least 10⁶ clusters per cm². In one embodiment, sequencing chemistries are employed having relatively high error rates. In such embodiments, the average quality scores produced by such chemistries are monotonically declining functions of sequence read lengths. In one embodiment, such decline corresponds to 0.5 percent of sequence reads have at least one error in positions 1-75; 1 percent of sequence reads have at least one error in positions 76-100; and 2 percent of sequence reads have at least one error in positions 101-125.

In one aspect, a sequence-based clonotype profile of an individual is obtained using the following steps: (a) obtaining a nucleic acid sample from T-cells and/or B-cells of the individual; (b) spatially isolating individual molecules derived from such nucleic acid sample, the individual molecules comprising at least one template generated from a nucleic acid in the sample, which template comprises a somatically rearranged region or a portion thereof, each individual molecule being capable of producing at least one sequence read; (c) sequencing said spatially isolated individual molecules; and (d) determining abundances of different sequences of the nucleic acid molecules from the nucleic acid sample to generate the clonotype profile. In one embodiment, each of the somatically rearranged regions comprise a V region and a J region. In another embodiment, the step of sequencing comprises bidirectionally sequencing each of the spatially isolated individual molecules to produce at least one forward sequence read and at least one reverse sequence read. Further to the latter embodiment, at least one of the forward sequence reads and at least one of the reverse sequence reads have an overlap region such that bases of such overlap region are determined by a reverse complementary relationship between such sequence reads. In still another embodiment, each of the somatically rearranged regions comprise a V region and a J region and the step of sequencing further includes determining a sequence of each of the individual nucleic acid molecules from one or more of its forward sequence reads and at least one reverse sequence read starting from a position in a J region and extending in the direction of its associated V region. In another embodiment, the step of sequencing comprises generating the sequence reads having monotonically decreasing quality scores. Further to the latter embodiment, monotonically decreasing quality scores are such that the sequence reads have error rates no better than the following: 0.2 percent of sequence reads contain at least one error in base positions 1 to 50, 0.2 to 1.0 percent of sequence reads contain at least one error in positions 51-75, 0.5 to 1.5 percent of sequence reads contain at least one error in positions 76-100. In another embodiment, the above method comprises the following steps: (a) obtaining a nucleic acid sample from T-cells of the individual; (b) spatially isolating individual molecules derived from such nucleic acid sample, the individual molecules comprising nested sets of templates each generated from a nucleic acid in the sample and each containing a somatically rearranged region or a portion thereof, each nested set being capable of producing a plurality of sequence reads each extending in the same direction and each starting from a different position on the nucleic acid from which the nested set was generated; (c) sequencing said spatially isolated individual molecules; and (d) determining abundances of different sequences of the nucleic acid molecules from the nucleic acid sample to generate the clonotype profile. In one embodiment, the step of sequencing includes producing a plurality of sequence reads for each of the nested sets. In another embodiment, each of the somatically rearranged regions comprise a V region and a J region, and each of the plurality of sequence reads starts from a different position in the V region and extends in the direction of its associated J region.

### Clonotype Determination from Sequence Data

Constructing clonotypes from sequence read data depends in part on the sequencing method used to generate such data, as the different methods have different expected read lengths and data quality. In one approach, a Solexa sequencer is employed to generate sequence read data for analysis as described in Faham and Willis (cited above). In one embodiment, a sample is obtained that provides at least 0.5-1.0x10⁶ lymphocytes to produce at least 1 million template molecules, which after optional amplification may produce a corresponding one million or more clonal populations of template molecules (or clusters). For most high throughput sequencing approaches, including the Solexa approach, such over sampling at the cluster level is desirable so that each template sequence is determined with a large degree of redundancy to increase the accuracy of sequence determination. For Solexa-based implementations, preferably the sequence of each independent template is determined 10 times or more. For other sequencing approaches with different expected read lengths and data quality, different levels of redundancy may be used for comparable accuracy of sequence determination. Those of ordinary skill in the art recognize that the above parameters, e.g. sample size, redundancy, and the like, are design choices related to particular applications.

In one aspect of the invention, sequences of clonotypes may be determined by combining information from one or more sequence reads, for example, along the V(D)J regions of the selected chains. In another aspect, sequences of clonotypes are determined by combining information from a plurality of sequence reads. Such pluralities of sequence reads may include one or more sequence reads along a sense strand (i.e. "forward" sequence reads) and one or more sequence reads along its complementary strand (i.e. "reverse" sequence reads). When multiple sequence reads are generated along the same strand, separate templates are first generated by amplifying sample molecules with primers selected for the different positions of the sequence reads. Such amplifications may be carried out in the same reaction or in separate reactions. In one aspect, whenever PCR is employed, separate amplification reactions are used for generating the separate templates which, in turn, are combined and used to generate multiple sequence reads along the same strand. This latter approach is preferable for avoiding the need to balance primer concentrations (and/or other reaction parameters) to ensure equal amplification of the multiple templates (sometimes referred to herein as "balanced amplification" or "unbias amplification").

### TCRβ Repertoire Analysis

In this example, TCRβ chains are analyzed. The analysis includes amplification, sequencing, and analyzing the TCRβ sequences. One primer is complementary to a common sequence in Cβ1 and Cβ2, and there are 34 V primers capable of amplifying all 48 V segments. Cβ1 or Cβ2 differ from each other at position 10 and 14 from the J/C junction. The primer for Cβ1 and Cβ2 ends at position 16 bp and has no preference for Cβ1 or Cβ2. The 34 V primers are modified from an original set of primers disclosed in Van Dongen et al, U.S. patent publication 2006/023423 4. The modified primers are disclosed in Faham et al, U.S. patent publication 2010/0151 471.

The Illumina Genome Analyzer is used to sequence the amplicon produced by the above primers. A two-stage amplification is performed on messenger RNA transcripts (1200), as illustrated in Figs. 1A-1B, the first stage employing the above primers and a second stage to add common primers for bridge amplification and sequencing. As shown in FIG. 1A, a primary PCR is performed using on one side a 20 bp primer (1202) whose 3' end is 16 bases from the J/C junction (1204) and which is perfectly complementary to Cβ1 (1203) and the two alleles of Cβ2. In the V region (1206) of RNA transcripts (1200), primer set (1212) is provided which contains primer sequences complementary to the different V region sequences (34 in one embodiment). Primers of set (1212) also contain a non-complementary tail (1214) that produces amplicon (1216) having primer binding site (1218) specific for P7 primers (1220). After a conventional multiplex PCR, amplicon (1216) is formed that contains the highly diverse portion of the J(D)V region (1206, 1208, and 1210) of the mRNA transcripts and common primer binding sites (1203 and 1218) for a secondary amplification to add a sample tag (1221) and primers (1220 and 1222) for cluster formation by bridge PCR. In the secondary PCR, on the same side of the template, a primer (1222 in Fig. 1B and referred to herein as "C10-17-P5") is used that has at its 3'end the sequence of the 10 bases closest to the J/C junction, followed by 17 bp with the sequence of positions 15-31 from the J/C junction, followed by the P5 sequence (1224), which plays a role in cluster formation by bridge PCR in Solexa sequencing. (When the C10-17-P5 primer (1222) anneals to the template generated from the first PCR, a 4 bp loop (position 11-14) is created in the template, as the primer hybridizes to the sequence of the 10 bases closest to the J/C junction and bases at positions 15-31 from the J/C junction. The looping of positions 11-14 eliminates differential amplification of templates carrying Cβ1 or Cβ2. Sequencing is then done with a primer complementary to the sequence of the 10 bases closest to the J/C junction and bases at positions 15-31 from the J/C junction (this primer is called C'). C10-17-P5 primer can be HPLC purified in order to ensure that all the amplified material has intact ends that can be efficiently utilized in the cluster formation.)

In FIG. 1A, the length of the overhang on the V primers (1212) is preferably 14 bp. The primary PCR is helped with a shorter overhang (1214). Alternatively, for the sake of the secondary PCR, the overhang in the V primer is used in the primary PCR as long as possible because the secondary PCR is priming from this sequence. A minimum size of overhang (1214) that supports an efficient secondary PCR was investigated. Two series of V primers (for two different V segments) with overhang sizes from 10 to 30 with 2 bp steps were made. Using the appropriate synthetic sequences, the first PCR was performed with each of the primers in the series and gel electrophoresis was performed to show that all amplified.

As illustrated in FIG. 1A, the primary PCR uses 34 different V primers (1212) that anneal to V region (1206) of RNA templates (1200) and contain a common 14 bp overhang on the 5' tail. The 14 bp is the partial sequence of one of the Illumina sequencing primers (termed the Read 2 primer). The secondary amplification primer (1220) on the same side includes P7 sequence, a tag (1221), and Read 2 primer sequence (1223) (this primer is called Read2_tagX_P7). The P7 sequence is used for cluster formation. Read 2 primer and its complement are used for sequencing the V segment and the tag respectively. A set of 96 of these primers with tags numbered 1 through 96 are created (see below). These primers are HPLC purified in order to ensure that all the amplified material has intact ends that can be efficiently utilized in the cluster formation.

As mentioned above, the second stage primer, C-10-17-P5 (1222, FIG. 1B) has interrupted homology to the template generated in the first stage PCR. The efficiency of amplification using this primer has been validated. An alternative primer to C-10-17-P5, termed CsegP5, has perfect homology to the first stage C primer and a 5' tail carrying P5. The efficiency of using C-10-17-P5 and CsegP5 in amplifying first stage PCR templates was compared by performing real time PCR. In several replicates, it was found that PCR using the C-10-17-P5 primer had little or no difference in efficiency compared with PCR using the CsegP5 primer.

Amplicon (1230) resulting from the 2-stage amplification illustrated in Figs. 1A-1C has the structure typically used with the Illumina sequencer as shown in FIG. 1C. Two primers that anneal to the outmost part of the molecule, Illumina primers P5 and P7 are used for solid phase amplification of the molecule (cluster formation). Three sequence reads are done per molecule. The first read of 100 bp is done with the C' primer, which has a melting temperature that is appropriate for the Illumina sequencing process. The second read is 6 bp long only and is solely for the purpose of identifying the sample tag. It is generated using a tag primer provided by the manufacturer (Illumina). The final read is the Read 2 primer, also provided by the manufacturer (Illumina). Using this primer, a 100 bp read in the V segment is generated starting with the 1 st PCR V primer sequence.

### EXAMPLE

In this example clonotype profiles were generated from each RNA sample from blood samples taken from AS patients and control individuals as indicated below. The method of generating clonotype profiles for TCRβs was essentially that described in Faham and Willis (cited above). After reverse transcription and two-staged PCR amplification as described above, sequences of the resulting amplicons were determined on an Illumina GA DNA sequencer using the manufacturer's suggested protocols. Each clonotype profile comprised about 2x10⁵ clonotypes constructed from about 1.3x10⁶ sequence reads generated from the Illumina sequencer. The clonotype profiles were analyzed to detect clonotypes or features of clonotypes that were shared among significant numbers of the AS patient samples but not the controls. It was discovered that a significant number of AS patients shared clonotypes that encoded the following peptide segments of TCRβs: LCASSLEASGSSYNEQFFGPGTRLTV (SEQ ID NO: 1) and VYFCASSDSSGSTDTQYFGPGTRLTV (SEQ ID NO: 2).

Clonotype profiles of control and AS patient samples were analyzed using conventional data mining techniques, e.g. Witten et al, Data Mining: Practical Machine Learning Tools and Techniques, Third Edition (Morgan Kaufman, 2011), with the objective of determining whether AS patients had clonotypes that encoded common amino acid sequence motifs. Sample sets from AS patients were set up as follows: (a) training was implemented on 56 patients positive for HLA B27 (1 sample/patient); (b) testing was implemented on 56 patients positive for HLA B27 (1 sample/patient); (c) confirmation was carried out on 57 samples from 16 patients (12 patients positive for HLA B27, 2 patients negative for HLA B27, and 2 patients with unknown HLA type). Control sample sets were set up as follows: (a) training was implemented on 521 samples from 120 lupus patients and 25 normal individuals; and (b) testing was carried out on 56 lupus patients (1 sample/patients, with samples matched on clonotype counts to AS test samples). Test and training samples from lupus patients were drawn from the same sample set but contained no overlapping patients. The training procedure examined a 26 amino acid sequence that spanned the TCRβ CDR3 region to determine shared amino acid sequences (i.e. putative functional clones) encoded by clonotypes of AS patients, but not controls. 374 putative functional clonotypes shared by at least 28 AS training samples were found. Searching for these clonotypes in the control training set found (a) 1 highly specific sequence (peptide 1)(seen in 5% of control samples and 12% of control individuals), (b) 1 moderately specific sequence (peptide 2)(seen in 15% of control samples and 27% of control individuals), and (c) all other sequences were seen in >18% of control samples and >37% of control individuals. In the test set, peptide 1 was present in 21/56 AS test samples versus 4/56 control samples (p value < 10⁻⁴) and peptide 2 was present in 29/56 AS test samples versus 13/56 control samples (p value < 10⁻³). In the confirmation set, peptide 1 was present in 14 samples from 6 patients, including 1 B27 positive patient, and peptide 2 was present in 36 samples from 10 patients, including both B27 positive patients.

### Definitions

Unless otherwise specifically defined herein, terms and symbols of nucleic acid chemistry, biochemistry, genetics, and molecular biology used herein follow those of standard treatises and texts in the field, e.g. Kornberg and Baker, DNA Replication, Second Edition (W.H. Freeman, New York, 1992); Lehninger, Biochemistry, Second Edition (Worth Publishers, New York, 1975); Strachan and Read, Human Molecular Genetics, Second Edition (Wiley-Liss, New York, 1999); Abbas et al, Cellular and Molecular Immunology, 6^{th} edition (Saunders, 2007).

"Aligning" means a method of comparing a test sequence, such as a sequence read, to one or more reference sequences to determine which reference sequence or which portion of a reference sequence is closest based on some sequence distance measure. An exemplary method of aligning nucleotide sequences is the Smith Waterman algorithm. Distance measures may include Hamming distance, Levenshtein distance, or the like. Distance measures may include a component related to the quality values of nucleotides of the sequences being compared.

"Amplicon" means the product of a polynucleotide amplification reaction; that is, a clonal population of polynucleotides, which may be single stranded or double stranded, which are replicated from one or more starting sequences. The one or more starting sequences may be one or more copies of the same sequence, or they may be a mixture of different sequences. Preferably, amplicons are formed by the amplification of a single starting sequence. Amplicons may be produced by a variety of amplification reactions whose products comprise replicates of the one or more starting, or target, nucleic acids. In one aspect, amplification reactions producing amplicons are "template-driven" in that base pairing of reactants, either nucleotides or oligonucleotides, have complements in a template polynucleotide that are required for the creation of reaction products. In one aspect, template-driven reactions are primer extensions with a nucleic acid polymerase or oligonucleotide ligations with a nucleic acid ligase. Such reactions include, but are not limited to, polymerase chain reactions (PCRs), linear polymerase reactions, nucleic acid sequence-based amplification (NASBAs), rolling circle amplifications, and the like, disclosed in the following references : Mullis et al, U.S. patents 4,683,195; 4,965,188; 4,683,202; 4,800,159 (PCR); Gelfand et al, U.S. patent 5,210,015 (real-time PCR with "taqman" probes); Wittwer et al, U.S. patent 6,174,670; Kacian et al, U.S. patent 5,399,491 ("NASBA"); Lizardi, U.S. patent 5,854,033; Aono et al, Japanese patent publ. JP 4-262799 (rolling circle amplification); and the like. In one aspect, amplicons of the invention are produced by PCRs. An amplification reaction may be a "real-time" amplification if a detection chemistry is available that permits a reaction product to be measured as the amplification reaction progresses, e.g. "real-time PCR" described below, or "real-time NASBA" as described in Leone et al, Nucleic Acids Research, 26: 2150-2155 (1998), and like references. As used herein, the term "amplifying" means performing an amplification reaction. A "reaction mixture" means a solution containing all the necessary reactants for performing a reaction, which may include, but not be limited to, buffering agents to maintain pH at a selected level during a reaction, salts, co-factors, scavengers, and the like.

"Antibody" or "immunoglobulin" means a protein, either natural or synthetically produced by recombinant or chemical means, that is capable of specifically binding to a particular antigen or antigenic determinant, which may be a target molecule as the term is used herein. Antibodies, e.g. IgG antibodies, are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intra-chain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Typically the binding characteristics, e.g. specificity, affinity, and the like, of an antibody, or a binding compound derived from an antibody, are determined by amino acid residues in the V_{H} and V_{L} regions, and especially in the CDR regions. The constant domains are not involved directly in binding an antibody to an antigen. Depending on the amino acid sequence of the constant domain of their heavy chains, immunoglobulins can be assigned to different classes. There are five major classes of immunoglobulins: IgA, IgD, IgE, IgG, and IgM, and several of these can be further divided into subclasses (isotypes), e.g., IgG, IgG₂, IgG₃, IgG₄, IgA₁, and IgA₂. "Antibody fragment", and all grammatical variants thereof, as used herein are defined as a portion of an intact antibody comprising the antigen binding site or variable region of the intact antibody, wherein the portion is free of the constant heavy chain domains (i.e. CH2, CH3, and CH4, depending on antibody isotype) of the Fc region of the intact antibody. Examples of antibody fragments include Fab, Fab', Fab'-SH, F(ab')₂, and Fv fragments; diabodies; any antibody fragment that is a polypeptide having a primary structure consisting of one uninterrupted sequence of contiguous amino acid residues (referred to herein as a "single-chain antibody fragment" or "single chain polypeptide"), including without limitation (1) single-chain Fv (scFv) molecules (2) single chain polypeptides containing only one light chain variable domain, or a fragment thereof that contains the three CDRs of the light chain variable domain, without an associated heavy chain moiety and (3) single chain polypeptides containing only one heavy chain variable region, or a fragment thereof containing the three CDRs of the heavy chain variable region, without an associated light chain moiety; and multispecific or multivalent structures formed from antibody fragments. The term "monoclonal antibody" (mAb) as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each mAb is directed against a single determinant on the antigen. In addition to their specificity, the monoclonal antibodies are advantageous in that they can be synthesized by hybridoma culture or by bacterial, yeast or mammalian expression systems, uncontaminated by other immunoglobulins. An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

"Clonality" as used herein means a measure of the degree to which the distribution of clonotype abundances among clonotypes of a repertoire is skewed to a single or a few clonotypes. Roughly, clonality is an inverse measure of clonotype diversity. Many measures or statistics are available from ecology describing species-abundance relationships that may be used for clonality measures in accordance with the invention, e.g. Chapters 17 & 18, in Pielou, An Introduction to Mathematical Ecology, (Wiley-Interscience, 1969). In one aspect, a clonality measure used with the invention is a function of a clonotype profile (that is, the number of distinct clonotypes detected and their abundances), so that after a clonotype profile is measured, clonality may be computed from it to give a single number. One clonality measure is Simpson's measure, which is simply the probability that two randomly drawn clonotypes will be the same. Other clonality measures include information-based measures and McIntosh's diversity index, disclosed in Pielou (cited above).

"Clonotype" means a recombined nucleotide sequence of a T cell encoding a T cell receptor (TCR), or a portion thereof. In one aspect, a collection of all the distinct clonotypes of a population of lymphocytes of an individual is a repertoire of such population, e.g. Arstila et al, Science, 286: 958-961 (1999); Yassai et al, Immunogenetics, 61: 493-502 (2009); Kedzierska et al, Mol. Immunol., 45(3): 607-618 (2008); and the like. As used herein, "clonotype profile," or "repertoire profile," is a tabulation of clonotypes of a sample of T cells (such as a peripheral blood sample containing such cells) that includes substantially all of the repertoire's clonotypes and their relative abundances. In one aspect of the invention, a clonotype comprises a nucleic acid that encodes a portion of a TCR β chain.

"Coalescing" means treating two candidate clonotypes with sequence differences as the same by determining that such differences are due to experimental or measurement error and not due to genuine biological differences. In one aspect, a sequence of a higher frequency candidate clonotype is compared to that of a lower frequency candidate clonotype and if predetermined criteria are satisfied then the number of lower frequency candidate clonotypes is added to that of the higher frequency candidate clonotype and the lower frequency candidate clonotype is thereafter disregarded. That is, the read counts associated with the lower frequency candidate clonotype are added to those of the higher frequency candidate clonotype.

"Complementarity determining regions" (CDRs) mean regions of an immunoglobulin (i.e., antibody) or T cell receptor where the molecule complements an antigen's conformation, thereby determining the molecule's specificity and contact with a specific antigen. T cell receptors and immunoglobulins each have three CDRs: CDR1 and CDR2 are found in the variable (V) domain, and CDR3 includes some of V, all of diverse (D) (heavy chains only) and joint (J), and some of the constant (C) domains.

"Effective amount" means an amount sufficient to ameliorate a symptom of an autoimmune condition. The effective amount for a particular patient may vary depending on such factors as the state of the autoimmune condition being treated, the overall health of the patient, method of administration, the severity of side-effects, and the like. Generally, a therapeutic antibody specific for an AS-related peptide is administered as a pharmaceutical composition comprising an effective amount of such antibody and a pharmaceutical carrier. A pharmaceutical carrier can be any compatible, non-toxic substance suitable for delivering the compositions of the invention to a patient. Generally, compositions useful for parenteral administration of such drugs are well known, e.g. Remington's Pharmaceutical Science, 15th Ed. (Mack Publishing Company, Easton, Pa. 1980). Alternatively, compositions of the invention may be introduced into a patient's body by implantable or injectable drug delivery system, e.g. Urquhart et al., Ann. Rev. Pharmacol. Toxicol., Vol. 24, pgs. 199-236 (1984); Lewis, ed. Controlled Release of Pesticides and Pharmaceuticals (Plenum Press, New York, 1981); U.S. Pat. No. 3,773,919; U.S. Pat. No. 3,270,960; and the like.

"Percent homologous," "percent identical," or like terms used in reference to the comparison of a reference sequence and another sequence ("comparison sequence") mean that in an optimal alignment between the two sequences, the comparison sequence is identical to the reference sequence in a number of subunit positions equivalent to the indicated percentage, the subunits being nucleotides for polynucleotide comparisons or amino acids for polypeptide comparisons. As used herein, an "optimal alignment" of sequences being compared is one that maximizes matches between subunits and minimizes the number of gaps employed in constructing an alignment. Percent identities may be determined with commercially available implementations of algorithms, such as that described by Needleman and Wunsch, J. Mol. Biol., 48: 443-453 (1970)("GAP" program of Wisconsin Sequence Analysis Package, Genetics Computer Group, Madison, WI), or the like. Other software packages in the art for constructing alignments and calculating percentage identity or other measures of similarity include the "BestFit" program, based on the algorithm of Smith and Waterman, Advances in Applied Mathematics, 2: 482-489 (1981) (Wisconsin Sequence Analysis Package, Genetics Computer Group, Madison, WI). In other words, for example, to obtain a polynucleotide having a nucleotide sequence at least 95 percent identical to a reference nucleotide sequence, up to five percent of the nucleotides in the reference sequence may be deleted or substituted with another nucleotide, or a number of nucleotides up to five percent of the total number of nucleotides in the reference sequence may be inserted into the reference sequence.

"Phage display" is a technique by which variant polypeptides are displayed as fusion proteins to at least a portion of a coat protein on the surface of phage, e.g., filamentous phage, particles. A utility of phage display lies in the fact that large libraries of randomized protein variants can be rapidly and efficiently selected for those sequences that bind to a target molecule with high affinity. Display of peptide and protein libraries on phage has been used for screening millions of polypeptides for ones with specific binding properties. Polyvalent phage display methods have been used for displaying small random peptides and small proteins through fusions to either gene III or gene VIII of filamentous phage. Wells and Lowman, Curr. Opin. Struct. Biol., 3:355-362 (1992), and references cited therein. In monovalent phage display, a protein or peptide library is fused to a gene III or a portion thereof, and expressed at low levels in the presence of wild type gene III protein so that phage particles display one copy or none of the fusion proteins. Avidity effects are reduced relative to polyvalent phage so that selection is on the basis of intrinsic ligand affinity, and phagemid vectors are used, which simplify DNA manipulations. Lowman and Wells, Methods: A companion to Methods in Enzymology, 3:205-0216 (1991).

"Polymerase chain reaction," or "PCR," means a reaction for the in vitro amplification of specific DNA sequences by the simultaneous primer extension of complementary strands of DNA. In other words, PCR is a reaction for making multiple copies or replicates of a target nucleic acid flanked by primer binding sites, such reaction comprising one or more repetitions of the following steps: (i) denaturing the target nucleic acid, (ii) annealing primers to the primer binding sites, and (iii) extending the primers by a nucleic acid polymerase in the presence of nucleoside triphosphates. Usually, the reaction is cycled through different temperatures optimized for each step in a thermal cycler instrument. Particular temperatures, durations at each step, and rates of change between steps depend on many factors well-known to those of ordinary skill in the art, e.g. exemplified by the references: McPherson et al, editors, PCR: A Practical Approach and PCR2: A Practical Approach (IRL Press, Oxford, 1991 and 1995, respectively). For example, in a conventional PCR using Taq DNA polymerase, a double stranded target nucleic acid may be denatured at a temperature >90°C, primers annealed at a temperature in the range 50-75°C, and primers extended at a temperature in the range 72-78°C. The term "PCR" encompasses derivative forms of the reaction, including but not limited to, RT-PCR, real-time PCR, nested PCR, quantitative PCR, multiplexed PCR, and the like. Reaction volumes range from a few hundred nanoliters, e.g. 200 nL, to a few hundred µL, e.g. 200 µL. "Reverse transcription PCR," or "RT-PCR," means a PCR that is preceded by a reverse transcription reaction that converts a target RNA to a complementary single stranded DNA, which is then amplified, e.g. Tecott et al, U.S. patent 5,168,03 8. "Real-time PCR" means a PCR for which the amount of reaction product, i.e. amplicon, is monitored as the reaction proceeds. There are many forms of real-time PCR that differ mainly in the detection chemistries used for monitoring the reaction product, e.g. Gelfand et al, U.S. patent 5,210,015 ("taqman"); Wittwer et al, U.S. patents 6,174,670 and 6,569,627 (intercalating dyes); Tyagi et al, U.S. patent 5,925,517 (molecular beacons) . Detection chemistries for real-time PCR are reviewed in Mackay et al, Nucleic Acids Research, 30: 1292-1305 (2002). "Nested PCR" means a two-stage PCR wherein the amplicon of a first PCR becomes the sample for a second PCR using a new set of primers, at least one of which binds to an interior location of the first amplicon. As used herein, "initial primers" in reference to a nested amplification reaction mean the primers used to generate a first amplicon, and "secondary primers" mean the one or more primers used to generate a second, or nested, amplicon. "Multiplexed PCR" means a PCR wherein multiple target sequences (or a single target sequence and one or more reference sequences) are simultaneously carried out in the same reaction mixture, e.g. Bernard et al, Anal. Biochem., 273: 221-228 (1999)(two-color real-time PCR). Usually, distinct sets of primers are employed for each sequence being amplified. Typically, the number of target sequences in a multiplex PCR is in the range of from 2 to 50, or from 2 to 40, or from 2 to 30. "Quantitative PCR" means a PCR designed to measure the abundance of one or more specific target sequences in a sample or specimen. Quantitative PCR includes both absolute quantitation and relative quantitation of such target sequences. Quantitative measurements are made using one or more reference sequences or internal standards that may be assayed separately or together with a target sequence. The reference sequence may be endogenous or exogenous to a sample or specimen, and in the latter case, may comprise one or more competitor templates. Typical endogenous reference sequences include segments of transcripts of the following genes: β-actin, GAPDH, β₂-microglobulin, ribosomal RNA, and the like. Techniques for quantitative PCR are well-known to those of ordinary skill in the art, as exemplified in the following references: Freeman et al, Biotechniques, 26: 112-126 (1999); Becker-Andre et al, Nucleic Acids Research, 17: 9437-9447 (1989); Zimmerman et al, Biotechniques, 21: 268-279 (1996); Diviacco et al, Gene, 122: 3013-3020 (1992); Becker-Andre et al, Nucleic Acids Research, 17: 9437-9446 (1989); and the like.

"Primer" means an oligonucleotide, either natural or synthetic that is capable, upon forming a duplex with a polynucleotide template, of acting as a point of initiation of nucleic acid synthesis and being extended from its 3' end along the template so that an extended duplex is formed. Extension of a primer is usually carried out with a nucleic acid polymerase, such as a DNA or RNA polymerase. The sequence of nucleotides added in the extension process is determined by the sequence of the template polynucleotide. Usually primers are extended by a DNA polymerase. Primers usually have a length in the range of from 14 to 40 nucleotides, or in the range of from 18 to 36 nucleotides. Primers are employed in a variety of nucleic amplification reactions, for example, linear amplification reactions using a single primer, or polymerase chain reactions, employing two or more primers. Guidance for selecting the lengths and sequences of primers for particular applications is well known to those of ordinary skill in the art, as evidenced by the following references : Dieffenbach, editor, PCR Primer: A Laboratory Manual, 2nd Edition (Cold Spring Harbor Press, New York, 2003).

"Quality score" means a measure of the probability that a base assignment at a particular sequence location is correct. A variety methods are well known to those of ordinary skill for calculating quality scores for particular circumstances, such as, for bases called as a result of different sequencing chemistries, detection systems, base-calling algorithms, and so on. Generally, quality score values are monotonically related to probabilities of correct base calling. For example, a quality score, or Q, of 10 may mean that there is a 90 percent chance that a base is called correctly, a Q of 20 may mean that there is a 99 percent chance that a base is called correctly, and so on. For some sequencing platforms, particularly those using sequencing-by-synthesis chemistries, average quality scores decrease as a function of sequence read length, so that quality scores at the beginning of a sequence read are higher than those at the end of a sequence read, such declines being due to phenomena such as incomplete extensions, carry forward extensions, loss of template, loss of polymerase, capping failures, deprotection failures, and the like.

"Repertoire", or "immune repertoire", or "immune receptor repertoire", means a set of distinct recombined nucleotide sequences, or clonotypes, that encode T cell receptors (TCRs) or fragments thereof, in a population of lymphocytes of an individual. Populations of lymphocytes from which a repertoire is determined may be taken from different tissue samples, to produce different immune repertoires. In some aspects of the invention, the population of lymphocytes corresponding to a repertoire may be circulating T cells, or may be subpopulations of the foregoing populations, including but not limited to, CD4+ T cells, or CD8+ T cells, or other subpopulations defined by cell surface markers, or the like. Such subpopulations may be acquired by taking samples from particular tissues, e.g. bone marrow, or lymph nodes, or the like, or by sorting or enriching cells from a sample (such as peripheral blood) based on one or more cell surface markers, size, morphology, or the like. In still other aspects, the population of lymphocytes corresponding to a repertoire may be derived from disease tissues, such as a tumor tissue, an infected tissue, or the like. In one embodiment, a repertoire comprising human TCR β chains or fragments thereof comprises a number of distinct nucleotide sequences in the range of from 0.1 x 10⁶ to 1.8 x 10⁶, or in the range of from 0.5 x 10⁶ to 1.5 x 10⁶, or in the range of from 0.8 x 10⁶ to 1.2 x 10⁶. In a particular embodiment, a repertoire of the invention comprises a set of nucleotide sequences encoding substantially all segments of the V(D)J region of TCRβ chain. In one aspect, "substantially all" as used herein means every segment having a relative abundance of .001 percent or higher; or in another aspect, "substantially all" as used herein means every segment having a relative abundance of .0001 percent or higher. In another embodiment, a repertoire of the invention comprises a set of nucleotide sequences having lengths in the range of from 25-200 nucleotides and including segments of the V, D, and J regions of a TCR β chain. In another embodiment, a repertoire of the invention comprises a number of distinct nucleotide sequences that is substantially equivalent to the number of lymphocytes expressing a distinct TCR β chain. In still another embodiment, "substantially equivalent" means that with ninety-nine percent probability a repertoire of nucleotide sequences will include a nucleotide sequence encoding an TCR β or portion thereof carried or expressed by every lymphocyte of a population of an individual at a frequency of .001 percent or greater. In still another embodiment, "substantially equivalent" means that with ninety-nine percent probability a repertoire of nucleotide sequences will include a nucleotide sequence encoding a TCR β or portion thereof carried or expressed by every lymphocyte present at a frequency of .0001 percent or greater. The sets of clonotypes described in the foregoing two sentences are sometimes referred to herein as representing the "full repertoire" of TCRβ sequences. As mentioned above, when measuring or generating a clonotype profile (or repertoire profile), a sufficiently large sample of lymphocytes is obtained so that such profile provides a reasonably accurate representation of a repertoire for a particular application. In one aspect, samples comprising from 10⁵ to 10⁷ lymphocytes are employed, especially when obtained from peripheral blood samples of from 1-10 mL.

"Sequence read" means a sequence of nucleotides determined from a sequence or stream of data generated by a sequencing technique, which determination is made, for example, by means of base-calling software associated with the technique, e.g. base-calling software from a commercial provider of a DNA sequencing platform. A sequence read usually includes quality scores for each nucleotide in the sequence. Typically, sequence reads are made by extending a primer along a template nucleic acid, e.g. with a DNA polymerase or a DNA ligase. Data is generated by recording signals, such as optical, chemical (e.g. pH change), or electrical signals, associated with such extension. Such initial data is converted into a sequence read. Typically, a clonotype is generated by coalescing multiple sequence reads.

"Sequence tree" means a tree data structure for representing nucleotide sequences. In one aspect, a tree data structure of the invention is a rooted directed tree comprising nodes and edges that do not include cycles, or cyclical pathways. Edges from nodes of tree data structures of the invention are usually ordered. Nodes and/or edges are structures that may contain, or be associated with, a value. Each node in a tree has zero or more child nodes, which by convention are shown below it in the tree. A node that has a child is called the child's parent node. A node has at most one parent. Nodes that do not have any children are called leaf nodes. The topmost node in a tree is called the root node. Being the topmost node, the root node will not have parents. It is the node at which operations on the tree commonly begin (although some algorithms begin with the leaf nodes and work up ending at the root). All other nodes can be reached from it by following edges or links.

## Claims

1. A method for determining a disease status of a patient suffering from, or suspected of suffering from, ankylosing spondylitis, the method comprising the steps of:
(a) amplifying molecules of nucleic acid from T-cells of a sample obtained from the patient, the molecules of nucleic acid comprising recombined DNA sequences from T-cell receptor genes;
(b) sequencing the amplified molecules of nucleic acid to form a clonotype profile;
(c) determining from the clonotype profile a presence, an absence and/or a level of clonotypes encoding segments of a T-cell receptor at least seventy percent identical to a segment in the group consisting of LCASSLEASGSSYNEQFFGPGTRLTV (SEQ ID NO: 1) and VYFCASSDSSGSTDTQYFGPGTRLTV (SEQ ID NO: 2); and
(d) correlating the presence, absence and/or level of such clonotypes with a status of ankylosing spondylitis in the patient.

2. The method of claim 1, wherein said step of determining comprises determining said level of said clonotypes encoding said segments of a T-cell receptor at least ninety percent identical to a segment in the group consisting of LCASSLEASGSSYNEQFFGPGTRLTV (SEQ ID NO: 1) and VYFCASSDSSGSTDTQYFGPGTRLTV (SEQ ID NO: 2), and wherein said step of correlating comprises correlating a presence or an elevated level of such clonotypes with ankylosing spondylitis in said patient.

3. The method of claim 1 or claim 2, wherein said sample is a peripheral blood sample.

4. A medication for ameliorating effects of ankylosing spondylititis for use in the treatment of a patient with an elevated level of clonotypes encoding segments of a T-cell receptor at least ninety percent identical to a segment in the group consisting of LCASSLEASGSSYNEQFFGPGTRLTV (SEQ ID NO: 1) and VYFCASSDSSGSTDTQYFGPGTRLTV (SEQ ID NO: 2), wherein the level of the clonotypes is determined by the method of claim 2.

5. The medication of claim 4 for use as defined in claim 4, wherein said medication is selected from the group consisting of an anti-inflammatory drug, a disease modifying antirheumatic drug (DMARD), and a TNFα blocker.

6. A medication for use in the treatment of a patient suffering from ankylosing spondylitis, wherein the disease status of the patient is determined by a method comprising the steps of:
determining in a clonotype profile of a tissue sample of the patient the presence, absence and/or quantity of clonotypes encoding segments of a T-cell receptor at least ninety percent identical to a segment in the group consisting of LCASSLEASGSSYNEQFFGPGTRLTV (SEQ ID NO: 1) and VYFCASSDSSGSTDTQYFGPGTRLTV (SEQ ID NO: 2); and
correlating a presence or an elevation in a level of such clonotypes to a presence of ankylosing spondylitis in the patient; and
wherein the medication is selected from the group consisting of an anti-inflammatory drug, a disease modifying anti-rheumatic drug (DMARD), and a TNFα blocker.

7. The medication of claim 6 for use as defined in claim 6, wherein said elevated level of said clonotype is at least .00001 percent of clonotypes in said clonotype profile.

8. The medication of claim 7 for use as defined in claim 7, wherein said elevated level of said clonotype is at least .0001 percent of clonotypes in said clonotype profile.

9. The medication of claim 8 for use as defined in claim 8, wherein said elevated level of said clonotype is at least .001 percent of clonotypes in said clonotype profile.

10. An isolated antibody specific for an amino acid segment of a T cell receptor, wherein the amino acid segment is selected from the group consisting of LCASSLEASGSSYNEQFFGPGTRLTV (SEQ ID NO: 1) and any 6 to 20 amino acid segment thereof and VYFCASSDSSGSTDTQYFGPGTRLTV (SEQ ID NO: 2) and any 6 to 20 amino acid segment thereof.

11. The antibody of claim 10 for use in the treatment of ankylosing spondylitis.

## Patentansprüche

1. Verfahren zur Bestimmung eines Erkrankungsstatus' eines Patienten, welcher an Spondylitis ankylosans leidet, oder bei dem vermutet wird, dass er an Spondylitis ankylosans leidet, wobei das Verfahren die Schritte umfasst:
(a) Amplifizieren von Nukleinsäuremolekülen aus T-Zellen einer Probe, welche von dem Patienten erhalten wurde, wobei die Nukleinsäuremoleküle rekombinante DNA-Sequenzen von T-Zellrezeptorgenen umfassen,
(b) Sequenzieren der amplifizierten Nukleinsäuremoleküle, um ein Klonotyp-Profil zu erstellen,
(c) Bestimmen aus dem Klonotyp-Profil ein Vorhandensein, ein Fehlen und/oder einen Spiegel von Klonotypen kodierend Segmente eines T-Zellrezeptors, welche wenigstens siebzig Prozent identisch mit einem Segment in der Gruppe bestehend aus LCASSLEASGSSYNEQFFGPGTRLTV (SEQ ID NO:1) und VYFCASSDSSGSTDTQYFGPGTRLTV (SEQ ID NO:2) sind, und
(d) Korrelieren des Vorhandenseins, des Fehlens und/oder des Spiegels von Klonotypen mit einem Status von Spondylitis ankylosans in dem Patienten.

2. Verfahren nach Anspruch 1, wobei der Schritt der Bestimmung eine Bestimmung des Spiegels der Klonotypen kodierend die Segmente eines T-Zellrezeptors umfasst, welche wenigstens neunzig Prozent identisch mit einem Segment in der Gruppe bestehend aus LCASSLEASGSSYNEQFFGPGTRLTV (SEQ ID NO:1) und VYFCASSDSSGSTDTQYFGPGTRLTV (SEQ ID NO:2) sind, und wobei der Schritt des Korrelierens die Korrelation eines Vorhandenseins oder eines erhöhten Spiegels eines solchen Klonotyps mit Spondylitis ankylosans in dem Patienten umfasst.

3. Verfahren nach Anspruch 1 oder Anspruch 2, wobei die Probe eine Probe aus peripherem Blut ist.

4. Medikation zur Linderung der Wirkungen von Spondylitis ankylosans zur Verwendung in der Behandlung eines Patienten mit einem erhöhten Spiegel von Klonotypen kodierend Segmente eines T-Rezeptors, welche wenigstens neunzig Prozent identisch mit einem Segment in der Gruppe bestehend aus LCASSLEASGSSYNEQFFGPGTRLTV (SEQ ID NO:1) und VYFCASSDSSGSTDTQYFGPGTRLTV (SEQ ID NO:2) sind, wobei der Spiegel der Klonotypen durch das Verfahren nach Anspruch 2 bestimmt wird.

5. Medikation nach Anspruch 4 zur Verwendung wie in Anspruch 4 definiert, wobei die Medikation ausgewählt ist aus der Gruppe bestehend aus einem antiinflammatorischen Wirkstoff, einem krankheitsmodifizierenden antirheumatischen Wirkstoff (DMARD) und einem TNFα-Blocker.

6. Medikation zur Verwendung in der Behandlung eines Patienten, welcher an Spondylitis ankylosans leidet, wobei der Erkrankungsstatus des Patienten durch ein Verfahren bestimmt wird, umfassend die Schritte:
Bestimmen in einem Klonotyp-Profil einer Gewebeprobe des Patienten das Vorhandensein, das Fehlen und/oder die Menge von Klonotypen kodierend Segmente eines T-Zellrezeptors, welche wenigstens neunzig Prozent identisch mit einem Segment in der Gruppe bestehend aus LCASSLEASGSSYNEQFFGPGTRLTV (SEQ ID NO:1) und VYFCASSDSSGSTDTQYFGPGTRLTV (SEQ ID NO:2) sind, und
Korrelieren eines Vorhandenseins oder einer Erhöhung eines Spiegels von solchen Klonotypen mit dem Vorhandensein von Spondylitis ankylosans in dem Patienten, und
wobei die Medikation ausgewählt ist aus der Gruppe bestehend aus einem antiinflammatorischen Wirkstoff, einem krankheitsmodifizierenden antirheumatischen Wirkstoff (DMARD) und einem TNFα-Blocker.

7. Medikation nach Anspruch 6 zur Verwendung wie in Anspruch 6 definiert, wobei der erhöhte Spiegel des Klonotyps wenigstens 0,00001 Prozent von Klonotypen in diesem Klonotyp-Profil ist.

8. Medikation nach Anspruch 7 zur Verwendung wie in Anspruch 7 definiert, wobei der erhöhte Spiegel des Klonotyps wenigstens 0,0001 Prozent von Klonotypen in diesem Klonotyp-Profil ist.

9. Medikation nach Anspruch 8 zur Verwendung wie in Anspruch 8 definiert, wobei der erhöhte Spiegel des Klonotyps wenigstens 0,001 Prozent von Klonotypen in diesem Klonotyp-Profil ist.

10. Isolierter Antikörper, welcher spezifisch für ein Aminosäuresegment eines T-Zellrezeptors ist, wobei das Aminosäuresegment ausgewählt ist aus der Gruppe bestehend aus LCASSLEASGSSYNEQFFGPGTRLTV (SEQ ID NO:1) und einem beliebigen 6- bis 20-Aminosäuresegment davon und VYFCASSDSSGSTDTQYFGPGTRLTV (SEQ ID NO:2) und einem beliebigen 6-bis 20-Aminosäuresegment davon.

11. Antikörper nach Anspruch 10 zur Verwendung in der Behandlung von Spondylitis ankylosans.

## Revendications

1. Procédé de détermination d'un état pathologique d'un patient atteint ou suspecté de souffrir d'une spondylarthrite ankylosante, le procédé comprenant les étapes consistant à :
(a) amplifier des molécules d'acide nucléique à partir de lymphocytes T d'un échantillon obtenu auprès du patient, les molécules d'acide nucléique comprenant des séquences d'ADN recombiné à partir de gènes récepteurs de lymphocytes T ;
(b) séquencer les molécules d'acide nucléique amplifiées pour former un profil de clonotype ;
(c) déterminer à partir du profil de clonotype une présence, une absence et/ou d'un niveau de clonotypes codant pour des segments d'un récepteur de lymphocytes T identique au moins à 70 % à un segment dans le groupe consistant en LCASSLEASGSSYNEQFFGPGTRLTV (SEQ ID NO:1) et VYFCASSDSSGSTDTQYFGPGTRLTV (SEQ ID NO:2) ; et
(d) mettre en corrélation la présence, l'absence et/ou le niveau de ces clonotypes avec un état de spondylarthrite ankylosante chez le patient.

2. Procédé selon la revendication 1, dans lequel ladite étape de détermination comprend la détermination dudit niveau de clonotypes codant pour lesdits segments d'un récepteur de lymphocytes T identique au moins à 90 % à un segment dans le groupe consistant en LCASSLEASGSSYNEQFFGPGTRLTV (SEQ ID NO:1) et VYFCASSDSSGSTDTQYFGPGTRLTV (SEQ ID NO:2) ; et dans lequel ladite étape de mise en corrélation comprend la mise en corrélation d'une présence ou d'un niveau élevé de telles que clonotypes avec une spondylarthrite ankylosante chez ledit patient.

3. Procédé selon la revendication 1 ou 2, dans lequel ledit échantillon est un échantillon de sang périphérique.

4. Médicament pour améliorer les effets de la spondylarthrite ankylosante, à utiliser dans le traitement d'un patient avec un niveau élevé de clonotypes codant pour des segments d'un récepteur de lymphocytes T identique au moins à 90 % à un segment dans le groupe consistant en LCASSLEASGSSYNEQFFGPGTRLTV (SEQ ID NO:1) et VYFCASSDSSGSTDTQYFGPGTRLTV (SEQ ID NO:2) ; dans lequel le niveau de clonotypes est déterminé par le procédé de la revendication 2.

5. Médicament selon la revendication 4 pour une utilisation selon la revendication 4, dans lequel ledit médicament est choisi dans le groupe consistant en un médicament anti-inflammatoire, un médicament antirhumatismal modificateur de la maladie (DMARD) et un inhibiteur de TNFα.

6. Médicament destiné à être utilisé dans le traitement d'un patient souffrant de spondylarthrite ankylosante, dans lequel l'état de la maladie du patient est déterminé par un procédé comprenant les étapes consistant à :
déterminer dans un profil de clonotype d'un échantillon de tissu du patient la présence, l'absence et/ou la quantité de clonotypes codant pour des segments d'un récepteur de lymphocytes T identique au moins à 90 % à un segment dans le groupe consistant en LCASSLEASGSSYNEQFFGPGTRLTV (SEQ ID NO:1) et VYFCASSDSSGSTDTQYFGPGTRLTV (SEQ ID NO:2) ; et
mettre en corrélation une présence ou une élévation d'un niveau de tels clonotypes avec la présence de la spondylarthrite ankylosante chez le patient ; et
dans lequel le médicament est choisi dans le groupe consistant en un médicament anti-inflammatoire, un médicament antirhumatismal modificateur de la maladie (DMARD) et un inhibiteur de TNFα.

7. Médicament selon la revendication 6 pour une utilisation selon la revendication 6, dans lequel ledit niveau élevé dudit clonotype est d'au moins 0,00001 % de clonotypes dans ledit profil de clonotype.

8. Médicament selon la revendication 7 pour une utilisation selon la revendication 7, dans lequel ledit niveau élevé dudit clonotype est d'au moins 0,0001 % de clonotypes dans ledit profil de clonotype.

9. Médicament selon la revendication 8 pour une utilisation selon la revendication 8, dans lequel ledit niveau élevé dudit clonotype est d'au moins 0,001 % de clonotypes dans ledit profil de clonotype.

10. Anticorps isolé spécifique pour un segment d'acides aminés d'un récepteur de lymphocyte T, dans lequel le segment d'acides aminés est choisi dans le groupe consistant en LCASSLEASGSSYNEQFFGPGTRLTV (SEQ ID NO:1) et tout segment de 6 à 20 acides aminés de celui-ci, et VYFCASSDSSGSTDTQYFGPGTRLTV (SEQ ID NO:2) et tout segment de 6 à 20 acides aminés de celui-ci.

11. Anticorps selon la revendication 10, destiné à être utilisé dans le traitement de la spondylarthrite ankylosante.
